# EUROPEAN PATENT SPECIFICATION

(11) **EP 3 885 444 B1**
(45) Date of publication and mention of the grant of the patent: **09.11.2022**
(21) Application number: 21275028.5
(22) Date of filing: 15.03.2021
(51) Int. Cl.: C12Q 1/04, G01N 33/569

(54) **A METHOD AND DEVICE FOR ENRICHING AND DETECTING MICROORGANISMS IN A BIOLOGICAL SAMPLE**
VERFAHREN UND VORRICHTUNG ZUR ANREICHERUNG UND ZUM NACHWEIS VON MIKROORGANISMEN IN EINER BIOLOGISCHEN PROBE
PROCÉDÉ ET DISPOSITIF PERMETTANT D'ENRICHIR ET DE DÉTECTER DES MICRO-ORGANISMES DANS UN ÉCHANTILLON BIOLOGIQUE

(30) Priority: 27.03.2020 CN 202010230413
(43) Date of publication of application: 29.09.2021
(73) Proprietor: Micronbrane Medical Co., Ltd., Taoyuan City, Taiwan 330007 (CN)
(72) Inventor: Chang, Yung, Taoyuan City, Taiwan, 338 (CN); Wu, Mengchu, Zhubei City Hsinchu County, Taiwan, 302 (CN); Jhong, Jheng-Fong, Kaohsiung City, Taiwan, 832 (CN); Chen, Yan-Wen, Kaohsiung City, Taiwan, 806 (CN); Hung, Hau, Zhubei City Hsinchu County , Taiwan, 302 (CN)
(74) Representative: Williams Powell

(56) References cited:
- EP-B1- 0 496 410
- CN-A- 110 339 732
- US-B2- 7 390 484

## Description

### Field of invention

The present invention relates to the technical field of detection of microorganisms; particularly, to a method and device for enriching and detecting microorganisms in a biological sample by reducing the interference from human-derived nucleated cells in the biological sample.

### Description of the related art

When using molecular detection methods to detect pathogenic microorganisms (including bacteria, mycoplasmas, fungi, viruses, spores etc.) in biological samples, the cell count and genome size of human cells in the samples are much larger than the pathogenic microorganisms, and the amount of human DNAs is usually tens of thousands or even millions of times that of DNAs of pathogenic microorganisms. Therefore, during the process of molecular detection of pathogenic microorganisms, the background interference of human DNAs has always been a major challenge. Currently, there are methods for differential lysis, such as QIAamp DNA Microbiome Kit and the recently published modified method (Nanopore metagenomic enables rapid clinical diagnosis of bacterial lower respiratory infection. Charalampous et al., Nature Biotechnology, 2019); as well as methylation modification methods to remove human DNAs, such as NEBNext^{®} Microbiome DNA Enrichment Kit (New England Biolabs). However, these methods have distinct disadvantages of complicated operations and inconsistent effects. EP0496410 B1 discloses the determination of bacteria by first filtering out the leukocytes and then capturing the bacteria on a second filter. CN 110339732 A discloses PVDF films as substrate grafted with a polymer of formula (I); e.g. polyvinylidene fluoride Ethylene-gN-hydroxyethyl acrylamide (PVDF-g-NHEMAA).

### Summary of the invention

Embodiments of the present invention provide a method and device for enriching and detecting microorganisms in a biological sample by reducing the interference from human-derived nucleated cells such as leukocytes in the biological sample.

In an embodiment, the present invention provides a method for enriching and detecting microorganisms in a biological sample, which includes the following steps: a) filtering the biological sample through a polymer-modified substrate, human-derived nucleated cells in the sample are captured or separated while the microorganisms in the sample pass or flow through the polymer-modified substrate into filtrate; and b) detecting the microorganisms present in the filtrate. The nucleated cells include one or more of erythroblasts, leukocytes and cancer cells, and the polymer is prepared by the polymerization of one or more monomers having the structure of the formula (1):

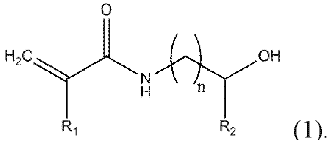

In formula (1), R₁ is independently selected from the group consisting of hydrogen, methyl, ethyl, hydroxyl, C₁₋₁₂ alkyl, phenyl; R₂ is independently selected from the group consisting of hydrogen, methyl, ethyl, C₁₋₆ alkyl, amino, phenyl; and n is an integer of 1 to 5.

Preferably, the microorganisms in the biological sample are bacteria.

Preferably, the microorganisms in the biological sample are fungi.

Preferably, the human-derived nucleated cells are leukocytes.

In some embodiments, the retention rate of the microorganisms in the filtrate is above 65%.

In some embodiments, the retention rate of the microorganisms in the filtrate is above 80%.

In some embodiments, the erythrocytes can pass or flow through the polymer-modified substrate into the filtrate, and the retention rate of the erythrocytes is above 80%.

In some embodiments, the platelets can pass or flow through the polymer-modified substrate into the filtrate, and the retention rate of the platelets is above 80%.

In some embodiments, the fibrinogens can pass or flow through the polymer-modified substrate into the filtrate, and the retention rate of the fibrinogens is above 80%.

In some embodiments, the detection rate of the microorganisms in the filtrate is 2 fold higher than the samples without filtration.

In some embodiments, the detection rate of the microorganisms in the filtrate is 40 fold higher than the sample without filtration.

In some embodiments, the monomer of formula (1) comprises N-Hydroxyethyl acrylamide, N-(2-Hydroxyethyl) acrylamide, NHEMAA, and N-(2-Hydroxyethyl) acrylamide, HEAA.

In some embodiments, the polymer further comprises an additional monomer, which may be butyl methacrylate, and the monomer of formula (1) is copolymerized with described additional monomer to form a copolymer.

In some embodiments, the polymer has the structure of formula (2):

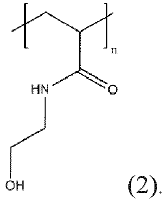

In formula (2), n is an integer of 10 to 50.

In some embodiments, the polymer has the structure of formula (4):

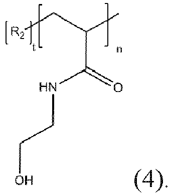

In formula (4), t is an integer of 50 to 90, n is an integer of 10 to 50, and R₂ is

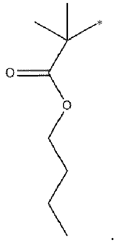

In some embodiments, the polymer is a segmented polymer.

In some embodiments, the polymer is disposed on the substrate in manners such as coating, spraying, or impregnating. The substrate includes, but is not limited to, polypropylene, polyethylene terephthalate, cellulose, polybutylene terephthalate. Elements of the surface of the modified substrate comprise carbon, oxygen, and nitrogen; the total mole percentage of carbon, oxygen, and nitrogen is defined as 100%, the mole percentage of carbon is from about 76.22% to 79.84%, the mole percentage of oxygen is from about 18.1% to 21.04%, and the mole percentage of nitrogen is from about 2.05% to about 2.75%.

In some embodiments, the filtrate is subjected to DNA purification, and is analyzed by PCR, qPCR, digital PCR, NGS, MassSpec, or Nanopore sequencing.

In some embodiments, the filtrate is subjected to DNA purification, a sequencing library is constructed by Oxford Nanopore rapid library construction process. Then, perform sequencing with Oxford Nanopore GridION sequencer.

In some embodiments, the biological samples is selected from the group consisting of blood, cerebral spinal fluid, cells, a cellular extract, a tissue sample, and a tissue biopsy.

In some embodiments, the method for enriching and detecting microorganisms in a biological sample according to the present invention can be used for pathogenic examination of biological samples.

In some embodiments, the invention also provides a device for enriching and detecting microorganisms in a biological sample. The device contains the following components: upper housing, filter, and lower housing. The filter is located between the upper housing and lower housing. The filter material contains the polymer-modified substrate as mentioned above; preferably, the filter is made from the polymer-modified substrate. The upper housing of the device may be provided with an inlet while the lower housing may be provided with an outlet. The biological sample enters the device from the inlet of the upper housing, penetrates through the filter, and flows out from the device through the outlet of the lower housing.

The method and device for enriching and detecting microorganisms in a biological sample provided by embodiments of the present invention enable the sample to be filtered through the Sterile Acrodisc^{®} White Blood Cell Syringe Filter (PALL) or polymer-modified substrate, which is highly specific in capturing or separating human-derived nucleated cells such as leukocytes. Besides, the microorganisms can pass through the Sterile Acrodisc^{®} White Blood Cell Syringe Filter (PALL) or polymer-modified substrate into the filtrate, thus enriching the microorganisms (including bacteria, mycoplasmas, fungi, viruses, spores etc.) in the sample during the reduction of human-derived nucleated cells. This process can therefore reduce the interference of the human cells in pathogenic examination.

In some embodiments, the method and device for enriching and detecting microorganisms in a biological sample comprises diagnosing sepsis in the individual.

The term "capture" and grammatical variations thereof means that the human-derived nucleated cells in the sample contact with the surface of the above substrate, and are attracted by the hydrophobic interactions, hydrogen bonding, or electrostatic intermolecular forces between the substrate and the cells, causing various types of human-derived nucleated cells to adhere directly to the surface of the modified substrate. The small size cells including fibrinogens and platelets may be attached before other larger cells. These processes are defined as "capture" of human-derived nucleated cells.

The term "separation" and grammatical variations thereof refers to the separation of human-derived nucleated cells from a sample after passing the sample containing human-derived nucleated cells through the material used to separates them. It also means that the content of human-derived nucleated cells in the sample can be reduced, or even significantly reduce. The process allows the concentration of human-derived nucleated cells in the resulting filtrate to be less than the original nucleated cells-containing sample.

The expression "reduction of nucleated cells" and grammatical variations thereof is not intended to mean that all or substantially all nucleated cells are completely removed. Instead, it is used to broadly indicate that the cell count of human-derived nucleated cells is reduced during separation or filtration.

### Brief Description of the Drawings

Embodiments of the present invention are described below, by way of example only, with reference to the accompanying drawings:
Figure 1: Schematic diagram of the process of an embodiment of the present invention for filtering a biological sample through a polymer-modified substrate.
Figure 2: The filtering device of an embodiment of the present invention for enriching and detecting microorganisms in a biological sample.
Figure 3: Structural formulas of the monomers and polymers of embodiments of the present invention, as well as theoretically predicted values of chemical shifts of nuclear magnetic resonance (NMR) spectrum signals thereof.
Figure 4: Measured map of NMR spectra of the monomers and polymers of embodiments of the present invention.
Figure 5: Coating density results of substrates of embodiments of the present invention, i.e., PP, PET, cellulose, and PBT modified with B-r-H and B-r-D.

### Symbol Description:

1. Upper housing; 2. Filter; 3. Lower housing.

### Detailed Description

Unless otherwise defined herein, scientific and technical terms used in connection with embodiments of the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well-known and commonly used in the art. Known methods and techniques are generally performed according to conventional methods well known in the art and as described in various general and more specific references that are discussed throughout the present specification unless otherwise indicated. Purification and DNA sequencing techniques are performed according to manufacturer's specifications, as commonly accomplished in the art or as described herein. The nomenclatures used in connection with the laboratory procedures and techniques described herein are those well-known and commonly used in the art.

In embodiments of the present invention, a biological sample can be filtered through a polymer-modified substrate The filter and modified substrate all have a highly specific human-derived nucleated cells capture or separation ability. Also, microorganisms in the biological sample can pass through the filter or polymer-modified substrate and enters filtrate. During the reduction of human-derived nucleated cells, a high level of microorganisms (bacteria, mycoplasmas, fungi, viruses, spores etc.) can be enriched in the sample, and thus reducing the interference from human-derived nucleated cells. The biological sample with effective enrichment of microorganisms can be subjected to DNA purification. A sequencing library is constructed with an appropriate concentration of the DNA purified sample according to Oxford Nanopore rapid library construction process, and it is sequenced using Oxford Nanopore GridION sequencer. Sequencing results show that Sterile Acrodisc^{®} White Blood Cell Syringe Filter (PALL) and the modified substrate can specifically remove human-derived nucleated cells and enrich microorganisms.

The Sterile Acrodisc^{®} White Blood Cell Syringe Filter used is from PALL (Catalog Nos. AP-4951 & AP-4952). The filter is a proven filtration device designed to separate leukocytes from whole blood samples while allowing red blood cells (RBCs) and platelets to flow through the membrane.

The method of enriching and detecting microorganisms in a biological sample according to an embodiment of the present invention includes: first, obtaining the biological sample, such as blood. Secondly, filtering the sample through a polymer-modified substrate. Human-derived nucleated cells in the sample are captured or separated. The content of nucleated cells in the obtained filtrate is greatly reduced. The microorganisms can be enriched in the filtrate by using the modified substrate during the reduction of human-derived nucleated cells.

The polymer of an embodiment of the present invention is prepared by the polymerization of one or more monomers having the structure of the formula (1):

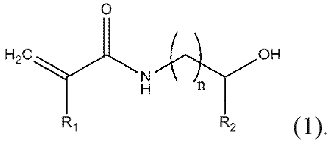

In formula (1), R₁ is independently selected from the group consisting of hydrogen, methyl, ethyl, hydroxyl, C₁₋₁₂ alkyl, and phenyl; R₂ is independently selected from the group consisting of hydrogen, methyl, ethyl, C₁₋₆ alkyl, amino, and phenyl; and n is an integer of 1 to 5.

According to some embodiments of the present invention, the R₁ in formula (1) is hydrogen, R₂ is hydrogen, and n is an integer of 1.

According to some embodiments of the present invention, the amide group and hydroxyl group-containing monomer is N-Hydroxyethyl acrylamide or N-(2-hydroxyethyl) acrylamide.

According to some embodiments of the present invention, the polymer is a copolymer prepared by the copolymerization of the monomer of formula (1) and at least one additional monomer, and the additional monomer is butyl methacrylate (BMA).

According to some embodiments of the present invention, the polymer is a segmented polymer.

According to some embodiments of the present invention, the polymer prepared by the monomer of formula (1) may have the structure of formula (2):

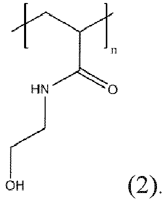

In formula (2), n is an integer of 10 to 50.

According to some embodiments of the present invention, the polymer prepared by the monomer of formula (1) may also have the structure of formula (4):

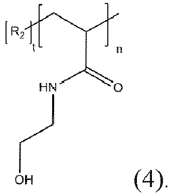

In formula (4), t is an integer of 50 to 90, n is an integer of 10 to 50.

R₂ is

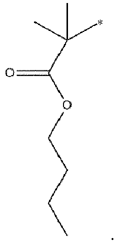

The preparation of the polymer is carried out by known technologies. For example, one monomer is used as the base end of the substrate, and another monomer is used as the functional end. The two monomers are mixed in a certain proportion, and follows with the addition of initiator ACVA. The solvent used for the preparation is ethanol. Next, the polymerization reaction is carried out at 70°C, thereby giving the desired polymer. After the reaction is completed, the product is precipitated using deionized water as precipitating agent and then dried.

The polymer of embodiments of the present invention can be coated, sprayed, or impregnated on the substrate to achieve the purpose of modifying the substrate. Specifically, dissolve an amount of polymer in ethanol as solvent to prepare a polymer solution; select a suitable substrate, cut it to a suitable size, and soak it in the polymer solution for about 1 minute; and then, wash the substrate surface with deionized water and dry. Accordingly, a surface-modified substrate coated with the polymer can be obtained. Materials used to manufacture the substrate can be polypropylene (PP), polyethylene terephthalate (PET), cellulose, polybutylene terephthalate (PBT), etc. Surface elements of the modified substrate include carbon, oxygen, and nitrogen, while the total mole percentage of them is defined as 100%. The mole percentage of carbon is about 76.22% to 79.84%, the mole percentage of oxygen is about 18.1% to 21.04%, and the mole percentage of nitrogen is about 2.05% to 2.75%.

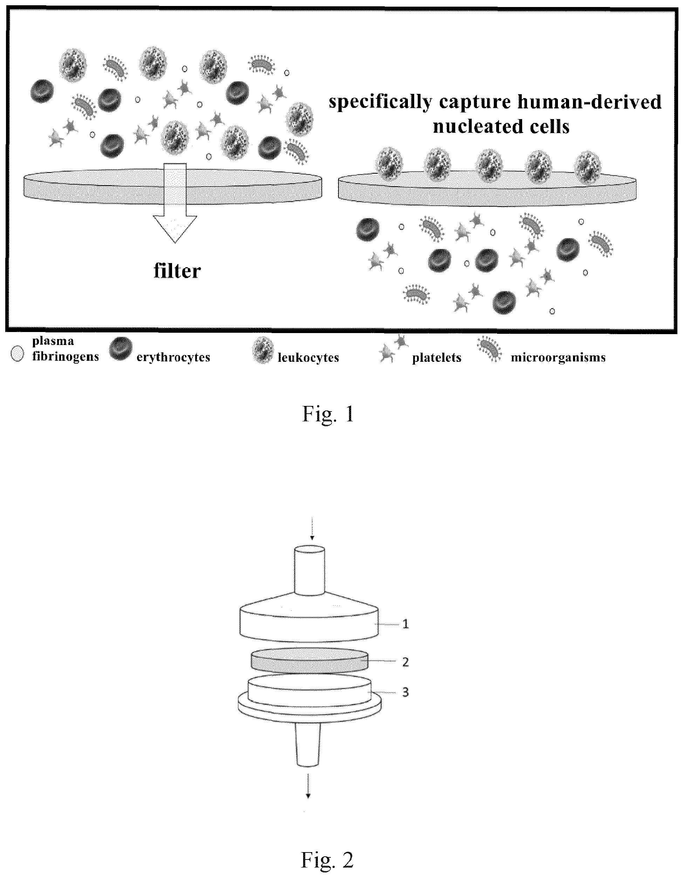

The biological sample is filtered through the polymer-modified substrate of an embodiment of the present invention. Refer to figure 1, which is a schematic diagram of the process of an embodiment of the present invention for filtering a biological sample through a modified substrate. The modified substrate can capture and absorb, attach or adhere human-derived nucleated cells (such as leukocytes), thus separating them from the sample such as whole blood. More importantly, the plasma proteins are hardly absorbed during the process of filtration while the platelet adhesion hardly occurs, which improves the retention rate of platelets. Besides, the rest of the biological sample (e.g., blood), such as erythrocytes, platelets, bacteria, viruses, and spores can pass/flow through the modified substrate to achieve the purification effect. The test results show that the removal rate of leukocytes in the filtered sample is greater than 70% and can even reach more than 90%. The microbial detection rate of the sample after filtration is at least twice that of the sample before filtration, and it can even reach a higher multiple, such as 40 folds .

Embodiments of the invention also provide a filtering device matched with the polymer modified substrate. As shown in figure 2, the device includes an upper housing 1, a filter 2, a lower housing 3. The filter 2 is located between the upper housing 1 and the lower housing 3. A sample inlet is provided on the upper housing 1, and a sample outlet is provided on the lower housing 3. The sample enters from the inlet of the upper housing 1 and penetrates through the filter 2. After that, it flows out through the outlet of the lower housing 3. The filter 2 is prepared from the polymer modified substrate described above.

Embodiments of methods for enriching and detecting microorganisms of the present invention can be applied to the filtering device. First, a biological sample such as blood is obtained; second, the sample is introduced from the inlet of the upper housing 1, filtered through the filter 2, and then flowed out through the lower housing 3. The human-derived nucleated cells such as leukocytes in the sample are captured or separated, and the content of nucleated cells in the filtrate is greatly reduced. The process enables to reduce the human-derived nucleated cells from the filtrate while allowing microorganisms to pass or flow through the filter 2. In addition, this process also enriches the microorganisms in the filtrate and reduces human-derived nucleated cells inference. The filtrate with effective enriched microorganisms is subjected to the process of DNA purification. DNA purified sample is used at an appropriate concentration, a sequencing library is constructed according to Oxford Nanopore rapid library construction process, and Oxford Nanopore GridION sequencer is used for sequencing. The sequencing result shows that the modified substrate can specifically remove human-derived nucleated cells' interference and achieve the goal of enrichment of microorganisms, leading to a great increase in the proportion of microorganisms.

The determination of DNA in embodiments of the present invention can be performed by common methods including PCR, qPCR, digital PCR, NGS, MassSpec, or Nanopore sequencing.

Oxford Nanopore sequencing technology used in Nanopore sequencing is the third generation single-molecule sequencing technology which has the advantages of simple sample handling, fast and long sequencing length (>10kbp) compared with NGS second-generation technology that requires amplified signals. These advantages are very suitable for the rapid identification of clinically unknown microbial pathogens. Therefore, this method can be applied to examine sepsis, detect pathogens and so on.

Embodiments of the present invention are further clarified below with reference to figures. It should be noted that the following embodiments provided are only for illustrative purpose and not intended to limit the invention.

### Experimental methods and materials

### Cell lines

The all cell lines were obtained from American Type Culture Collection (ATCC). The TF1 (ATCC^{®} CRL-2003^{™}) and Jurkat clone E6-1 (ATCC^{®} TIB-152^{™}) were cultured in RPMI medium with 10% FBS, PC-3 (ATCC^{®} CRL1435^{™}) was cultured in F12K medium with 10% FBS, SK-BR-3 (ATCC^{®} HTB30^{™}) was cultured in McCoy's 5A (modified) medium with 10% FBS, and K-562 (ATCC^{®} CCL243^{™}) was cultured in IMDM (Iscove's Modified Dulbecco's Medium) with 10% FBS in incubator at 37□, 5% CO₂.

### 1. Experimental materials for substrate modification

The material of inventive monomer is N-Hydroxyethyl acrylamide (HEAA), which has both a hydroxyl functional group and an amide functional group, and has a chemical structure as follows:

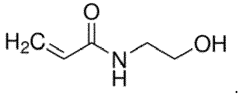

The material of comparative monomer is positively charged N,N-dimethylaminoethyl methacrylate (DMAEMA), which has the following chemical structure:

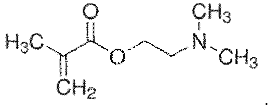

Besides, butyl methacrylate (BMA) is used as the base end of the polymer of the present embodiment of the invention, so that the polymer can be physically absorbed on the surface of substrate. BMA has the following chemical structure:

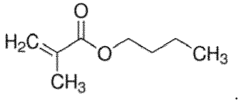

4,4'-azobis(4-cyanovaleric acid) (ACVA) is used as initiator, and has the following chemical structure:

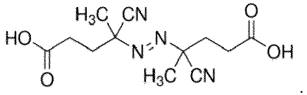

The materials of the substrate include polypropylene (PP), polyethylene terephthalate (PET), cellulose, polybutylene terephthalate (PBT). The chemical structures are respectively as follows:

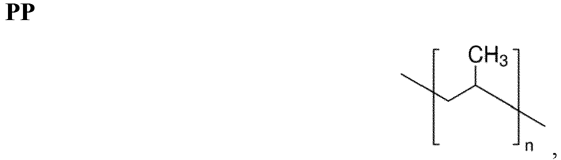

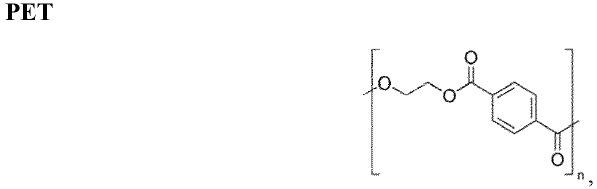

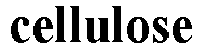

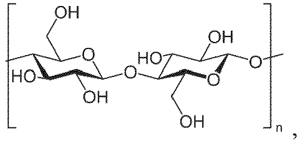

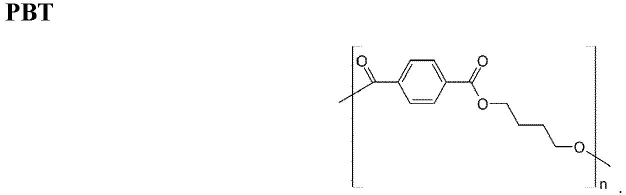

### 2. Preparation of polymers for substrate modification

BMA as the base end and HEAA or DMAEMA as the functional end were mixed according to the given proportion (about 70% of the base end and about 30% of the functional end), and were added ACVA as initiator, and ethanol as solvent. Next, the polymerization reaction was carried out at 70°C for 24 hours. BMA-r-HEAA and BMA-r-DMAEMA polymers could be prepared respectively. After the reaction was completed, the product was precipitated using deionized water as precipitating agent and then dried.

### 3. Surface modification of the substrate

Each of PP, PET, cellulose, and PBT was selected as the substrate to be modified.

### 3.1 PP substrate modification by physical adsorption

BMA-r-HEAA polymer and BMA-r-DMAEMA polymer were each taken and formulated into a polymer solution using ethanol as a solvent. A PP substrate was taken and cut to a suitable size, soaked in the BMA-r-HEAA polymer solution for 1 minute, then the residual solution on the surface was washed off with deionized water, and then the PP substrate was dried; and similar operations as described above were done starting with a PP substrate and the BMA-r-DMAEMA polymer solution. Accordingly, surface-modified PP substrate coated with BMA-r-HEAA polymer and surface-modified PP substrate coated with BMA-r-DMAEMA polymer can be obtained respectively.

### 3.2 PET substrate modification by physical adsorption

BMA-r-HEAA polymer and BMA-r-DMAEMA polymer were each taken and formulated into a polymer solution using ethanol as a solvent. A PET substrate was taken and cut to a suitable size, soaked in the BMA-r-HEAA polymer solution for 1 minute, then the residual solution on the surface was washed off with deionized water, and then the PET substrate was dried; and similar operations as described above were done starting with a PET substrate and the BMA-r-DMAEMA polymer solution. Accordingly, surface-modified PET substrate coated with BMA-r-HEAA polymer and surface-modified PET substrate coated with BMA-r-DMAEMA polymer can be obtained respectively.

### 3.3 Cellulose substrate modification by physical adsorption

BMA-r-HEAA polymer and BMA-r-DMAEMA polymer were each taken and formulated into a polymer solution using ethanol as a solvent. A cellulose substrate was taken and cut to a suitable size, soaked in the BMA-r-HEAA polymer solution for 1 minute, then the residual solution on the surface was washed off with deionized water, and then the cellulose substrate was dried; and similar operations as described above were done starting with a cellulose substrate and the BMA-r-DMAEMA polymer solution. Accordingly, surface-modified cellulose substrate coated with BMA-r-HEAA polymer and surface-modified cellulose substrate coated with BMA-r-DMAEMA polymer can be obtained respectively.

### 3.4 PBT substrate modification by physical adsorption

BMA-r-HEAA polymer and BMA-r-DMAEMA polymer were each taken and formulated into a polymer solution using ethanol as a solvent. A PBT substrate was taken and cut to a suitable size, soaked in the BMA-r-HEAA polymer solution for 1 minute, then the residual solution on the surface was washed off with deionized water, and then the PBT substrate was dried; and similar operations as described above were done starting with a PBT substrate and the BMA-r-DMAEMA polymer solution. Accordingly, surface-modified PBT substrate coated with BMA-r-HEAA polymer and surface-modified PBT substrate coated with BMA-r-DMAEMA polymer can be obtained respectively.

### 3.5 Identification of nuclear magnetic resonance (NMR)

10 mg of each of the above polymers was weighted and dissolved in 1 mL of methanol (d-MeOH) to give a solution with a concentration of 10 mg/mL, respectively. The solution was placed in an NMR test tubes, and delivered to the Instrumentation Center of National Central University in Taiwan for performing measurements. Then the chemical structures and the monomer ratios of the polymers were analyzed and calculated by the characteristic peaks of the spectra.

### 3.6 Density measurements of surface modification

Before modification, the PP, PET, cellulose, and PBT substrates were weighted by a microbalance. After the surfaces were modified completely, the substrates were dried, and the weights of the surface-modified substrates were measured via a microbalance. The weight of the polymer modified on the surface of the substrate, in each case, can be obtained by calculating the difference in weights of the substrate before and after the modification. Finally, the weight of the polymer per unit area, i.e., the surface coating density, can be obtained through conversion.

### 3.7 Blood filtration test

The modified PP, PET, cellulose, and PBT substrates were each cut into a circle with a diameter of 2.6 cm, and 20 layers of the substrates were staked. Then, the substrates were placed and locked in a filtering device (similar to the device shown in Figure 2). This part of the experiment is divided into two methods:
Method 1: 10 mL of whole blood was filtered through the modified substrate in the filter. Then, the blood sample before and after the filtration were examined using hemocytometer to calculate the leukocyte removal rate and platelet retention rate.
Method 2: 1 mL of *E. coli* liquid (6 × 10⁹ cells/ mL) was added to 9 mL of blood sample, and shaken for 5 minutes in a blood test tube mixer. Then, the blood sample was taken out and filtrated. Mixtures containing blood sample and other bacteria (such as *Staphylococcus aureus*) or fungi (such as *Aspergillus brasiliense*) were also prepared in the same way.

### 3.8 Enzyme linked immunosorbent assay (ELISA)

The blood samples obtained before and after the filtration of method 1 and method 2 from embodiment 3.7 were centrifuged using a centrifuge respectively. The supernatant (i.e., plasma) was extracted and diluted 10-fold with PBS. After dilution, 0.25 mL of the sample was taken out and transferred to a 24-well plate (24 well-tissue culture polystyrene plate, 24-well TCPS plate). To the sample was added 0.25 mL of the first antibody specific for Fibrinogen (Monoclonal Anti-human Fibrinogen, Clone, Sigma Aldrich Co., cat: F4639), and placed in oven at 37□ for 30 minutes. 0.25 mL of secondary antibody (anti-mouse IgG, rabbit IgG whole, HRP conjugated, Wako Co., cat: 014-1761) was added, which is specific for the first antibody and will specifically bind to the first antibody. After being placed in a 37□ oven for 30 minutes, 0.25 mL of a developer (3,3',5,5'-Tetramethylbenzidine, TMB) was added, waiting 6 minutes for color development, and then 0.25 mL of 1M sulfuric acid was added to each sample to stop the reaction. 200 µl of solution from each sample (including TCPS empty wells) was pipetted into a 96-well plate and analyzed at a UV wavelength of 450 nm by using Bio-tek model PowerWare XS microplate reader. After obtaining the UV readings for the samples before and after filtration, the protein recovery ratio can be obtained.

### 3.9 Microbial filtration test

Use a variety of different microorganisms, including fungi and Gram (-) and Gram (+) bacteria, such as *Aspergillus brasiliensis*, *E. Coli*, *Shewanella algae* (SA), *Staphylococcus pseudintermedius* (SP), *Staphylococcus aureus, Klebsiella pneumoniae* (CRKP), *lmtechella halotolerans* (*I. Halo*), and *Allobacillus halotolerans* (*A. Halo*) etc. The same amount of microbial liquid was filtered or not filtered. The Sterile Acrodisc^{®} White Blood Cell Syringe Filter (PALL), modified PP, PET, cellulose, and PBT substrates were used to test the capacity of microbial filtration.

Group 1: *Shewanella algae* (Gram-), *Staphylococcus pseudintermedius* (Gram-), and *Klebsiella pneumoniae* (Gram-).

Group 2: *E. coli* (Gram-), *Staphylococcus aureus* (Gram+), and *Aspergillus brasiliensis* (fungi).

Group 3: *Imtechella halotolerans* (Gram-) and *Allobacillus halotolerans* (Gram+).

### 3.10 DNA purification, concentration, and quantitative sequencing

The blood samples added with bacteria prepared under method 2 of embodiment 3.7 were divided into two, one was filtered, and the other was not filtered as a control. The samples were each centrifuged and the supernatant (i.e. plasma) was extracted. The supernatants and bacterial filtration samples of embodiment 3.9 were each subjected to DNA purification by DNeasy Blood and Tissue Kit. The concentration of purified DNA was measured by nanodrop spectrophotometer for comparison of the concentrations before and after filtration, and analyzed for the corresponding bacteria or fungi by Semi-quantitative Real-Time PCR. An appropriate concentration of DNA was taken and a sequencing library was constructed according to the Oxford Nanopore rapid library construction process. The sequencing results of Oxford Nanopore GridION sequencing proves that the modified substrates remove the host leukocytes and human DNAs, and enrich the microbial DNAs in the samples.

### 4. The detection results of substrate modification

### 4.1 Analysis of Nuclear magnetic resonance (NMR)

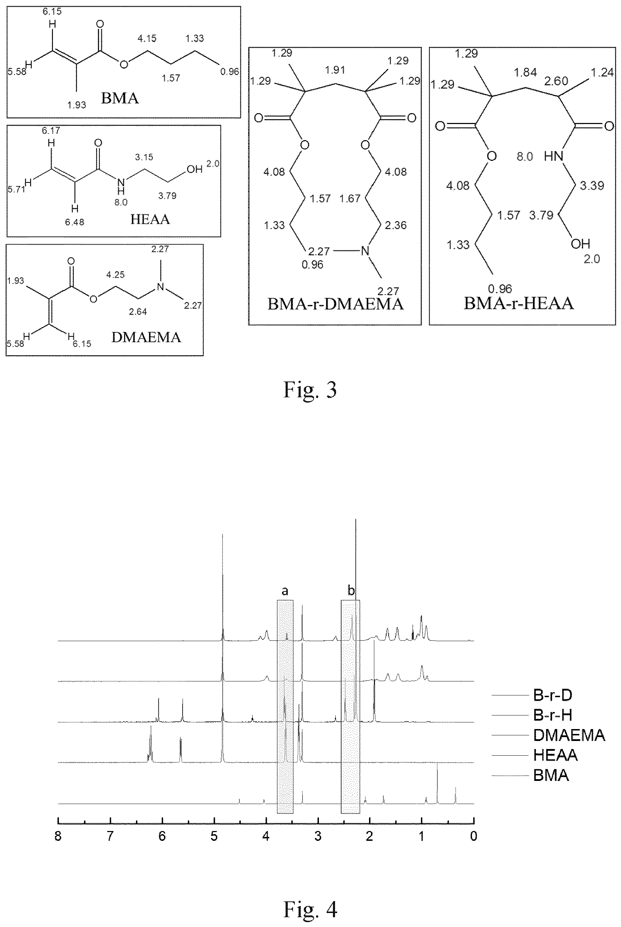

As shown in Table 1 below, for the convenience of further description, the polymer compounds such as BMA-r-HEAA and BMA-r-DMAEMA are abbreviated as B-r-H and B-r-D respectively. Figure 3 shows the structural formulas and their theoretically predicted values of chemical shift of each one of the monomer compounds and polymer compounds. Figure 4 shows the measured map of NMR spectra of monomer and polymer structures. ¹H NMR (300 MHz, DMSO-*d*₆) δ 13.21 (s, 1H), 10.25 (s, 1H), 9.10 (s, 1H), 8.66 (s, 1H), 8.25 (s, 1H), 8.12 (s, 1H), 8.10 -7.82 (m, 5H), 7.17 (d, J = 8.7 Hz, 1H), 7.14 (t, J = 74.7 Hz, 1H), 3.77- 3.73 (m, 4H), 3.04- 3.76 (m, 4H). The specific experimental conditions and actual test values are listed in Table 1. According to the NMR analysis, the characteristic peak of HEAA is mainly appeared at site "a", and the characteristic peak of DMAEMA is mainly appeared at site "b". It is shown according to the NMR spectra that the polymer compounds used in this experiment have been successfully synthesized.

**Table 1**

| **Polymer/monomer name** | **Abbreviation** | **NMR test condition and data** |
|---|---|---|
| BMA-r-HEAA | B-r-H | ¹H NMR (600 MHz, Methanol-*d*₄) δ 3.99 (s, 2H), 3.61 (s, 1H), 1.97 (s, 2H), 1.66 (s, 3H), 1.46 (s, 3H), 0.99 (s, 6H), 0.90 (s, 1H). |
| BMA-r-DMA | B-r-D | ¹H NMR (600 MHz, Methanol-*d*₄) δ 4.12 (s, 1H), 3.99 (s, 2H), 2.66 (s, 1H), 2.35 (d, *J* = 10.3 Hz, 3H), 1.99 - 1.94 (m, 2H), 1.90 - 1.86 (m, 1H), 1.67 (s, 3H), 1.48 (s, 3H), 1.08 (s, 2H), 1.01 (s, 4H), 0.91 (s, 3H). |
| DMAEMA | DMAEMA | ¹H NMR (600 MHz, Methanol-*d*₄) δ 6.07 (dt, *J* = 2.4, 1.2 Hz, 2H), 5.64 - 5.58 (m, 2H), 4.84 (d, *J* = 13.5 Hz, 1H), 3.65 (td, *J* = 6.2, 2.0 Hz, 4H), 2.48 (t, *J* = 6.1 Hz, 4H), 2.33 - 2.24 (m, 5H), 2.27 (s, 10H), 1.93 (dt, *J* = 7.4, 1.4 Hz, 7H). |
| HEAA | HEAA | ¹H NMR (600 MHz, Methanol-*d*₄) δ 6.07 (s, 1H), 5.60 (q, *J* = 1.7 Hz, 1H), 4.15 (td, *J* = 6.6, 1.4 Hz, 2H), 1.92 (s, 3H), 1.70 - 1.62 (m, 2H), 1.47 - 1.38 (m, 2H), 0.96 (td, *J* = 7.4, 1.3 Hz, 3H). |
| BMA | BMA | ¹H NMR (600 MHz, Methanol-*d*₄) δ 6.30 -6.17 (m, 2H), 5.64 (dt, *J* = 9.9, 2.1 Hz, 1H), 3.62 (td, *J* = 5.8, 1.7 Hz, 2H), 3.37 (td, *J* = 5.8, 1.8 Hz, 2H). |

### 4.2 Coating density measurement results of PP, PET, cellulose, and PBT substrates

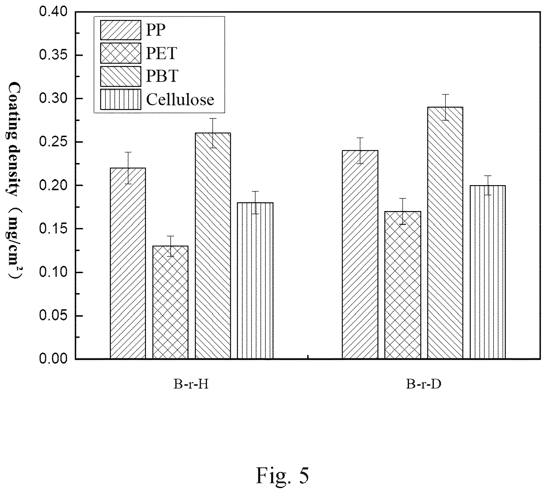

Figure 5 shows the coating density results of PP, PET, cellulose, and PBT substrates each modified with any of B-r-H and B-r-D. By respectively weighing the weights of the substrates before and after modification as well as measuring the surface areas of the substrates, the coating density of each polymer on the surface of each of the substrates can be calculated, and the densities were ranging from about 0.1 to about 0.3 mg/cm².

### 4.3 Filtration test results of blood cells

The modified substrate is used to filter blood as well as blood added with bacteria as described for blood filtration test above. The content of various blood cells before and after filtering in the blood samples can be determined by a hemocytometer. Accordingly, the leukocyte removal rate and the retention rate of each of various blood cells can be calculated. Fibrinogen was detected by ELISA, and bacteria such as *E. Coli* are detected using an absorption spectrometer.

RBC is referred to red blood cell (erythrocyte), WBC is referred to white blood cell (leukocyte), PLT is referred to platelet, *E. coli* is referred to as *Escherichia coli*, and Fibrinogen is referred to plasma fibrinogen. As shown in Table 2, the substrates modified by HEAA have a leukocyte capture rate of at least 94%, and retains more than 93% of erythrocytes, more than 87% of platelets, more than 93% of plasma fibrinogens. Thus, when whole blood flows through the modified substrate, most of the leukocytes will adhere to the substrate while most of the erythrocytes, platelets, and plasma fibrinogens can be retained in the filtrate. Accordingly, the B-r-H modified substrate of the present embodiment of the invention is high specific in capturing leukocytes without capturing or attaching erythrocytes and platelets. Thus, it is considered as a good material for leukocyte reduction for whole blood sample. Certainly, in other embodiments, for instance, an erythrocyte concentrate or platelet concentrate can also be allowed to penetrate through the above-mentioned modified substrate to specifically remove leukocytes.

The method of capturing, separating, or filtering leukocytes using substrate modified by B-r-H not only increases the capture rate of leukocytes, but also retains most of the erythrocytes, platelets, and plasma fibrinogens. Therefore, it is obvious that the method of filtering blood through substrate modified by B-r-H is effected by a different mechanism of blood filtration compared to the method of filtering leukocytes through substrate modified by polymer such as B-r-D and DMAEMA.

Table 3 shows the result of filtration after adding *E. Coli* to the blood. The result shows that the HEAA-modified substrate has a leukocyte capture rate of at least 95%, and retains more than 92% of the erythrocytes, more than 87% of platelets. Besides, the retention rate of the *E. Coli* is more than 82%. This means that when whole blood flows through the modified substrate, most of the leukocytes will adhere to the substrate, and the remaining filtrate can retain other cells and bacteria in the blood. Accordingly, the B-r-H modified substrate of the present embodiment of the invention has a very high specificity for leukocyte capture compared to the conventional method of capturing, separating, or filtering leukocytes through substrate modified with polymers such as B-r-D and DMAEMA. In addition, the B-r-H modified substrate will not capture or attach erythrocytes and platelets. It can retain most of the erythrocytes, platelets, and plasma fibrinogens in the filtrate, and most of the bacteria can pass through the modified substrate without attachment. Thus, when using the filtrate with leukocytes specifically removed, the accuracy and efficiency of pathogenic examination can be effectively improved.

**Table 2**

| | | RBC (10⁶/µL) | WBC (10³/µL) | PLT (10³/µL) | RBC retention rate | WBC removal rate | PLT retention rate | Fibrinogen retention rate |
|---|---|---|---|---|---|---|---|---|
| before filtration | | 3.75 | 6.54 | 263 | - | - | - | 100.00% |
| **polymer** | **substrate** | | | | | | | |
| B-r-H | PP | 3.53 | 0.27 | 231 | 94.13% | 95.87% | 87.83% | 93.06% |
| | PET | 3.54 | 0.25 | 229 | 94.40% | 96.18% | 87.07% | 96.07% |
| | PBT | 3.52 | 0.35 | 233 | 93.87% | 94.65% | 88.59% | 94.95% |
| | Cellulose | 3.55 | 0.32 | 235 | 94.67% | 95.11% | 89.35% | 96.31% |
| B-r-D | PP | 3.46 | 0.16 | 111 | 92.27% | 97.55% | 42.21% | 45.84% |
| | PET | 3.47 | 0.24 | 119 | 92.53% | 96.33% | 45.25% | 47.90% |
| | PBT | 3.46 | 0.23 | 114 | 92.27% | 96.48% | 43.35% | 46.39% |
| | Cellulose | 3.44 | 0.27 | 109 | 91.73% | 95.87% | 41.44% | 50.75% |

**Table 3**

| | | RBC (10⁶/_{µL}) | WBC (1 0³/_{µL}) | PLT (10³/µL) | RBC retention rate | WBC removal rate | PLT retention rate | E.Coil OD₆₀₀ | E.coil retention rate | Fibrinogen retention rate |
|---|---|---|---|---|---|---|---|---|---|---|
| before filtration | | 3.47 | 6.20 | 221 | - | - | - | 0.784 | - | 100.00% |
| **polymer** | **substrate** | | | | | | | | | |
| B-r-H | PP | 3.25 | 0.31 | 194 | 93.66% | 95.00% | 87.78% | 0.667 | 85.08% | 95.71% |
| | PET | 3.29 | 0.23 | 196 | 94.81% | 96.29% | 88.69% | 0.650 | 82.91% | 96.72% |
| | PBT | 3.20 | 0.30 | 193 | 92.22% | 95.16% | 87.33% | 0.656 | 83.67% | 95.16% |
| | Cellulose | 3.26 | 0.25 | 198 | 93.95% | 95.97% | 89.59% | 0.659 | 84.06% | 94.06% |
| B-r-D | PP | 3.25 | 0.31 | 100 | 93.66% | 95.00% | 45.25% | 0.254 | 32.40% | 46.43% |
| | PET | 3.26 | 0.30 | 99 | 93.95% | 95.16% | 44.80% | 0.241 | 30.74% | 49.39% |
| | PBT | 3.23 | 0.32 | 101 | 93.08% | 94.84% | 45.70% | 0.272 | 34.69% | 45.44% |
| | Cellulose | 3.27 | 0.28 | 97 | 94.24% | 95.48% | 43.89% | 0.254 | 32.40% | 48.16% |

### 4.4 DNA concentration results of bacterial filtration test

According to the bacterial filtration test method mentioned above, group 1 of bacteria were subjected to filtration test using B-r-H modified substrate, which is highly specific for leukocytes capture and can retain other cells as well as bacteria. Table 4 shows data of the identical amount of bacterial solution, including *Shewanella algae* (SA), *Staphylococcus pseudintermedius* (SP), *Klebiella pneumoniae* (CRKP). After filtering through B-r-H modified substrate, the amount of DNA recovered in the filtered sample can reach more than 92% of the sample without filtering. This result indicates that most of the common bacteria are allowed to penetrate through the highly specific modified substrate.

**Table 4**

| Bacteria ID | Filtrate Vol. (ul) | % of V Loss | DNA conc. (ng/ul) from 1 ml filtrate | DNA conc. (ng/ul) from 1 ml, control | DNA Recovery (%) |
|---|---|---|---|---|---|
| SA (Gram -) | 1730 | 13.5 | 3.76 | 4.07 | 92.38 |
| SP (Gram +) | 1640 | 18.0 | 1.86 | 1.7 | 109.41 |
| CRKP (Gram -) | 1670 | 16.5 | 3.07 | 2.82 | 108.87 |

### 4.5 Semi-quantitative Real-Time PCR semi-quantitative comparison results of blood simulation samples

Group 2 of microorganisms were subjected to the test. 10⁶ CFU/mL or 10⁴ CFU/mL of mixed microorganisms (*E. Coli*: *Staphylococcus aureus*: *Aspergillus brasiliensis* = 1:1:1) was added to normal human whole blood. 5 mL of the sample was centrifuged at 2,000 g for 15 mins, the upper plasma was removed and filtered with a B-r-H modified substrate or unfiltered. The sample was then centrifuged at 13,500 rpm for 10 mins to remove the supernatant and DNA of the bottom product was purified. The purified DNAs were subjected to a semi-quantitative comparison using primers (table 5) of specific bacteria, according to SYBR-green Semi-quantitative Real-Time PCR semi-quantitative method on Light Cycler 96 (Roche) PCR instrument. The semi-quantitative analysis results were shown in Table 6. W/O filter is referred to unfiltered samples, while W/ filter is referred to filtered samples. The corresponding concentrations of *E. coli* (Gram-), *Staphylococcus aureus* (Gram +), and *Aspergillus brasiliensis* (fungi) in blood samples filtered through B-r-H modified substrates and in unfiltered blood samples were not significantly different. The results show that after filtering through B-r-H modified substrate, most of the bacteria and fungi in the blood samples can penetrate through this substrate highly specific in leukocyte capture.

**Table 5**

| **SEQ ID No.** | **Primer Name** | **Primer sequence** |
|---|---|---|
| 1 | *E. coli*-F | GTCCAAAGCGGCGATTTG |
| 2 | *E. coli*-R | CCAGCCATGCACACTGATAC |
| 3 | *S. aureus*-F | AAGCGCATAACAAGCGAGAT |
| 4 | *S. aureus*-R | TGACGGAATGCATTTGATGT |
| 5 | *A. brasiliensis*_18S-F | CTGAAAGCGTGCAGTCTGAG |
| 6 | *A. brasiliensis_*18S-R | TGTGCGTTCAAAGACTCGAT |

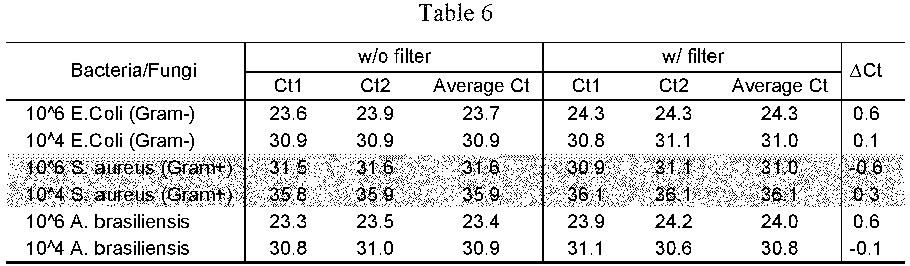

### 4.6 Metagenomic sequencing results of blood simulation samples

### 4.6.1 In situ positive control test:

ZymoBIOMICS^{™} Spike-in Control I (High Microbial Load) (ZYMO RESEARCH, Catalog Nos. D6320 & D6320-10) was used as an *in situ* positive control, which consists of equal cell numbers of two bacteria strains, *Imtechella halotolerans* and *Allobacillus halotolerans.* Normal human whole blood samples were added with 1×10⁴ and 1×10⁶ cells/mL of total bacteria, respectively. 5 mL of the sample was centrifuged at 2,000g for 15 mins, the upper plasma was removed and filtered with Sterile Acrodisc^{®} White Blood Cell Syringe Filter (PALL), B-r-H modified substrate, or unfiltered, respectively. Then, the sample was centrifuged at 13,500 rpm for 10 mins to remove the supernatant and DNA in the bottom product was purified by TANBead^{®} Nucleic Acid Extraction kit Blood Bacterial DNA Auto Plate (TAN Bead, CatM6BGA45). A sequencing library was constructed by Oxford Nanopore Technologies (ONT) SQK-RBK004 Rapid Barcoding Kit, and the purified DNAs were sequenced using ONT FLOMIN106 (revD) flow cell. The sequence results were shown in Table 7. W/O is referred to unfiltered samples, and W/f Devin and W/f PALL are referred to B-r-H modified substrate and Sterile Acrodisc^{®} White Blood Cell Syringe Filter (PALL) filtered samples, respectively. ΔA and ΔI refer to that when the human cell is the basic value, whether the relatively amount of bacteria is greater than human cell. As the ΔA and ΔI value is lower, means the more the relatively amount of bacteria. The results show that after filtering through Sterile Acrodisc^{®} White Blood Cell Syringe Filter (PALL) or B-r-H modified substrate, it can indeed enrich the microorganisms in the blood samples. Among them, the testing group 1×10⁴ cells/mL has the most significant effect. The concentration of pathogenic microorganisms in the general human body is also quite low, so the result is in line with our expectations.

**Table 7**

| | ***I. Halo*** | ***A. Halo*** | **Homo** | **ΔI** | **ΔΔI** | **ΔA** | **ΔΔA** |
|---|---|---|---|---|---|---|---|
| **1 × 10⁴ cells/mL** | | | | | | | |
| **w/o** | 27.6 | 26.22 | 28.58 | -0.98 | - | -2.36 | - |
| **w/f Devin** | 30.05 | 26.46 | 33.47 | -3.42 | -2.44 | -7.01 | -4.65 |
| **w/f PALL** | 28.06 | 26.12 | 33.04 | -4.98 | -4.00 | -6.92 | -4.56 |

| **1 × 10⁶ cells/mL** | | | | | | | |
|---|---|---|---|---|---|---|---|
| **w/o** | 20.23 | 18.66 | 28.99 | -8.76 | - | -10.33 | - |
| **w/f Devin** | 21.4 | 20.49 | 31.1 | -9.70 | -0.94 | -10.61 | -0.28 |
| **w/f PALL** | 21.22 | 19.19 | 31.16 | -9.94 | -1.18 | -11.97 | -1.64 |

### 4.6.2 Pathogenic microorganisms test:

Normal human whole blood was added with 2.5× 10³ CFU/mL of *E. coli* and 1 ng/mL of *Klebsiella pneumonia* gDNA. 5 mL of the sample was centrifuged at 2,000g for 15 mins, the upper plasma was removed and filtered with a B-r-H modified substrate or unfiltered. Then, the sample was centrifuged at 13,500 rpm for 10 mins to remove the supernatant and DNA of the bottom product was purified. A sequencing library was constructed by Oxford Nanopore Technologies (ONT) SQK-RBK004 Rapid Barcoding Kit, and the purified DNAs were sequenced using ONT FLOMIN106 (revD) flow cell. The sequence results were shown in Table 8. W/O filter-1 and W/O filter-2 are referred to unfiltered samples, and W/ filter-1 and W/ filter-2 are referred to filtered samples. The percentages of *K. Pneumonia* in the samples filtered through B-r-H modified substrate increased from 0.2% and 0.3% to 10.6% and 13.4% compared to unfiltered samples, respectively. The percentages of *E. coli* increased from 0.05% and 0.03% to 2.8% and 5.2%, respectively. The results show that after filtering through B-r-H modified substrate, it can indeed enrich the microorganisms in the blood samples and increase the detection rate of microorganisms by more than 40 folds.

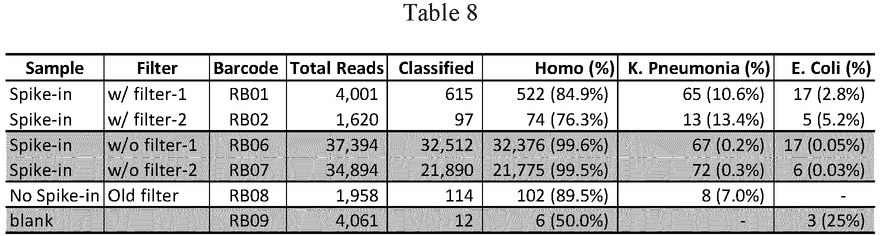

### 5. Filtration test of human-derived nucleated cells

Five human nucleated cell lines (see Table 9) were used to test the human-derived nucleated cells capture ability of the B-r-H modified substrate. The cells were harvested and resuspended with 5 mL PBS, then took 10 µL cells for counting. After counting, the cells were diluted into 1 × 10⁶ cells/ mL and make the final volume of the samples (1 × 10⁶ cells/ mL in PBS) > 3 mL. Then, 20 µL of samples were took and counted three times with LUNA-II Automated Cell Counter (Logos Biosystems). Added 1 mL of sample into the syringe barrel and filtrated through the B-r-H modified substrate. Finally, took 20 µL of the filtrate for cell counting (3 repeats).

The results show that the modified substrate has a nucleated cell capture rate of at least 99%. This means that when biological samples flow through the modified substrate, most of the human-derived nucleated cells will adhere to the substrate. The results are show in Table 10. The human nucleated cells in the samples are specifically removed after filtered through B-r-H modified substrate.

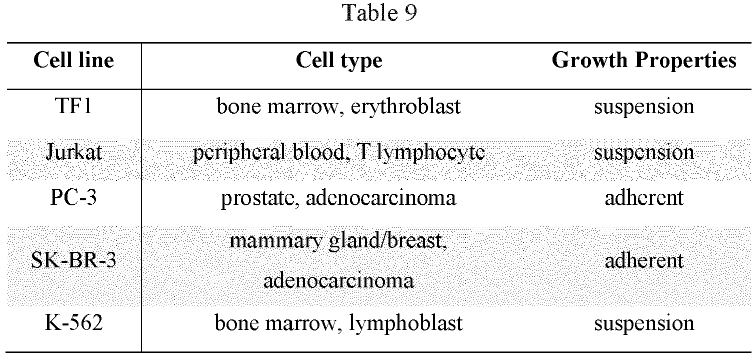

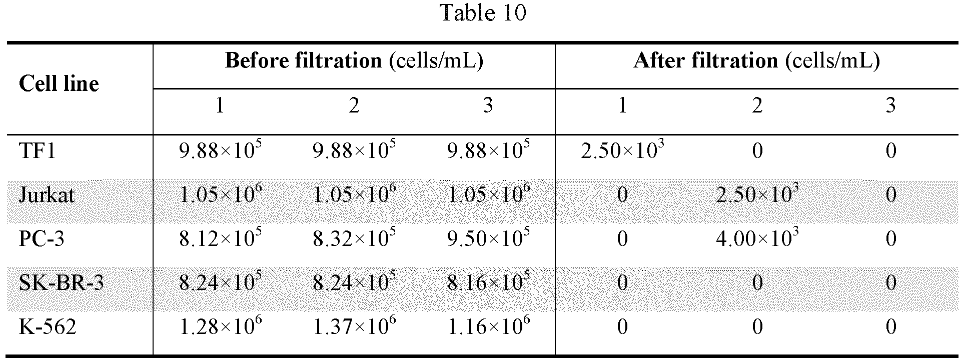

## Claims

1. A method for enriching and detecting microorganisms in a biological sample, comprising the following steps: a) filtering the biological sample through a polymer-modified substrate, wherein human-derived nucleated cells in the sample are captured or separated by the polymer-modified substrate and the microorganisms in the sample pass or flow through the polymer-modified substrate into filtrate; and b) detecting the microorganisms present in the filtrate; wherein the nucleated cells include one or more of erythroblasts, leukocytes and cancer cells; and the polymer is prepared by the polymerization of one or more monomers having the structure of formula (1): 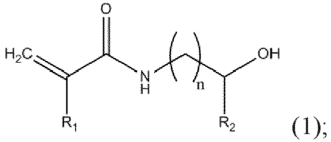 wherein R₁ is independently selected from the group consisting of hydrogen, methyl, ethyl, hydroxyl, C₁₋₁₂ alkyl, phenyl; R₂ is independently selected from the group consisting of hydrogen, methyl, ethyl, C₁₋₆ alkyl, amino, phenyl; and n is an integer of 1 to 5.

2. The method of claim 1, wherein the human-derived nucleated cells are leukocytes.

3. The method of claim 1, wherein the microorganisms are bacteria.

4. The method of claim 1, wherein the microorganisms are fungi.

5. The method of any preceding claim, wherein erythrocytes in the sample pass or flow through the polymer-modified substrate into the filtrate.

6. The method of any of claims 1 to 4, wherein platelets in the sample pass or flow through the polymer-modified substrate into the filtrate.

7. The method of any of claims 1 to 4, wherein fibrinogens in the sample pass or flow through the polymer-modified substrate into the filtrate.

8. The method of any preceding claim, wherein the monomer of formula (1) is N-hydroxyethyl acrylamide, N-(2-hydroxyethyl) acrylamide, NHEMAA, and N-(2-Hydroxyethyl)acrylamide, HEAA.

9. The method of any preceding claim, wherein the polymer further comprises an additional monomer, which is butyl methacrylate, and the monomer of formula (1) is copolymerized with the additional monomer to form a copolymer.

10. The method of any preceding claim, wherein the polymer has the structure of formula (2): 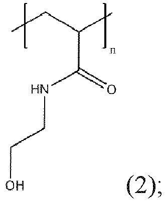 wherein n is an integer of 10 to 50.

11. The method of any preceding claim, wherein the polymer has the structure of formula (4): 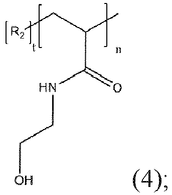 wherein t is an integer of 50 to 90, n is an integer of 10 to 50, and R₂ is

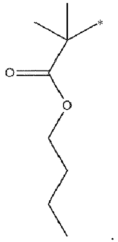

12. The method of any preceding claim, wherein the polymer is a segmented polymer.

13. The method of any preceding claim, wherein the polymer is disposed on the substrate by coating, spraying, or impregnating.

14. The method of any preceding claim, wherein the substrate is polypropylene, polyethylene terephthalate, cellulose, polybutylene terephthalate.

15. The method of any preceding claim, wherein surface elements of the modified substrate comprise carbon, oxygen, and nitrogen; the total mole percentage of carbon, oxygen, and nitrogen is defined as 100%, the mole percentage of carbon is about 76.22% to 79.84%, the mole percentage of oxygen is about 18.1% to 21.04%, and the mole percentage of nitrogen is about 2.05% to 2.75%.

16. The method of any preceding claim, wherein the method is used for pathogenic examination of biological samples.

17. The method of any preceding claim, wherein the biological sample is selected from the group consisting of blood, cerebral spinal fluid, cells, a cellular extract, a tissue sample, and a tissue biopsy.

18. The method of any preceding claim, wherein the filtrate is subjected to DNA purification, and is analyzed by PCR, qPCR, digital PCR, NGS, MassSpec, or Nanopore sequencing.

19. The method of any preceding claim, wherein the filtrate is subjected to DNA purification, a sequencing library is constructed by Oxford Nanopore rapid library construction process, and it is sequenced with Oxford Nanopore GridION sequencer.

20. A device for use in the method of any preceding claim, comprising: upper housing, filter, and lower housing; wherein the filter is located between the upper housing and lower housing, and is made from the polymer-modified substrate, wherein the polymer of the polymer-modified substrate is prepared by the polymerization of one or more monomers having the structure of formula (1): 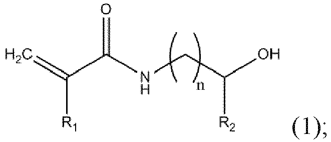 wherein R₁ is independently selected from the group consisting of hydrogen, methyl, ethyl, hydroxyl, C₁₋₁₂ alkyl, phenyl; R₂ is independently selected from the group consisting of hydrogen, methyl, ethyl, C₁₋₆ alkyl, amino, phenyl; and n is an integer of 1 to 5.

21. The device of claim 20, wherein the upper housing of the device is provided with an inlet while the lower housing is provided with an outlet; the biological sample enters the device from the inlet of the upper housing, penetrates through the filter, and flows out from the device through the outlet of the lower housing.

22. Use of a device as claimed in claim 20 or 21, to prepare biological samples for pathogenic examination.

## Patentansprüche

1. Verfahren zur Anreicherung und zum Nachweis von Mikroorganismen in einer biologischen Probe, das die folgenden Schritte umfasst: a) Filtrieren der biologischen Probe durch ein polymermodifiziertes Substrat, wobei vom Menschen stammende einen Zellkern aufweisende Zellen in der Probe durch das polymermodifizierte Substrat eingefangen oder abgetrennt werden und die Mikroorganismen in der Probe durch das polymermodifizierte Substrat in das Filtrat gelangen oder fließen; und b) Nachweisen der in dem Filtrat vorhandenen Mikroorganismen; wobei die einen Zellkern aufweisenden Zellen zumindest Erythroblasten, Leukozyten und/oder Krebszellen aufweisen; und das Polymer durch die Polymerisation von einem oder mehreren Monomeren mit der Struktur der Formel (1) hergestellt wird: 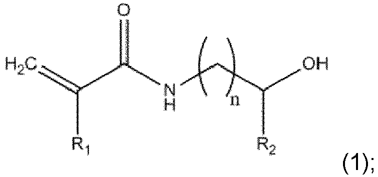 wobei R₁ unabhängig aus der Gruppe bestehend aus Wasserstoff, Methyl, Ethyl, Hydroxyl, C1-12-Alkyl, Phenyl ausgewählt ist; R2 unabhängig aus der Gruppe bestehend aus Wasserstoff, Methyl, Ethyl, C1-6-Alkyl, Amino, Phenyl ausgewählt ist; und n eine ganze Zahl von 1 bis 5 ist.

2. Verfahren nach Anspruch 1, wobei die vom Menschen stammenden einen Zellkern aufweisenden Zellen Leukozyten sind.

3. Verfahren nach Anspruch 1, wobei die Mikroorganismen Bakterien sind.

4. Verfahren nach Anspruch 1, wobei die Mikroorganismen Pilze sind.

5. Verfahren nach einem der vorhergehenden Ansprüche, wobei die Erythrozyten in der Probe das polymermodifizierte Substrat passieren oder durch dieses in das Filtrat fließen.

6. Verfahren nach einem der vorhergehenden Ansprüche, wobei Blutplättchen in der Probe durch das polymermodifizierte Substrat in das Filtrat gelangen oder fließen.

7. Verfahren nach einem der Ansprüche 1 bis 4, wobei Fibrinogene in der Probe durch das polymermodifizierte Substrat in das Filtrat gelangen oder fließen.

8. Verfahren nach einem der vorhergehenden Ansprüche, wobei das Monomer der Formel (1) N-Hydroxyethylacrylamid, N-(2-Hydroxyethyl)acrylamid, NHEMAA, und N-(2-Hydroxyethyl)acrylamid, HEAA, ist.

9. Verfahren nach einem der vorhergehenden Ansprüche, wobei das Polymer weiter ein zusätzliches Monomer umfasst, das Butylmethacrylat ist, und das Monomer der Formel (1) mit dem zusätzlichen Monomer copolymerisiert wird, um ein Copolymer zu bilden.

10. Verfahren nach einem der vorhergehenden Ansprüche, wobei das Polymer die Struktur der Formel (2) aufweist: 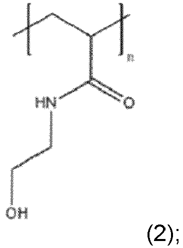 wobei n eine ganze Zahl von 10 bis 50 ist.

11. Verfahren nach einem der vorhergehenden Ansprüche, wobei das Polymer die Struktur der Formel (4) aufweist: 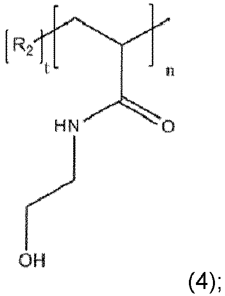 worin t eine ganze Zahl von 50 bis 90 ist, n eine ganze Zahl von 10 bis 50 ist und R₂ 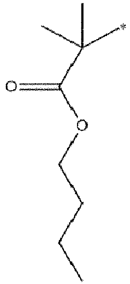 ist.

12. Das Verfahren nach einem der vorhergehenden Ansprüche, wobei das Polymer ein segmentiertes Polymer ist.

13. Verfahren nach einem der vorhergehenden Ansprüche, wobei das Polymer durch Beschichten, Besprühen oder Imprägnieren auf das Substrat aufgebracht wird.

14. Verfahren nach einem der vorhergehenden Ansprüche, wobei das Substrat Polypropylen, Polyethylenterephthalat, Cellulose oder Polybutylenterephthalat ist.

15. Verfahren nach einem der vorhergehenden Ansprüche, wobei die Oberflächenelemente des modifizierten Substrats Kohlenstoff, Sauerstoff und Stickstoff umfassen; wobei der Gesamtmolprozentsatz von Kohlenstoff, Sauerstoff und Stickstoff als 100% definiert ist, der Molprozentsatz von Kohlenstoff etwa 76,22% bis 79,84% beträgt, der Molprozentsatz von Sauerstoff etwa 18,1% bis 21,04% beträgt und der Molprozentsatz von Stickstoff etwa 2,05% bis 2,75% beträgt.

16. Verfahren nach einem der vorhergehenden Ansprüche, wobei das Verfahren zur pathogenen Untersuchung von biologischen Proben verwendet wird.

17. Verfahren nach einem der vorhergehenden Ansprüche, wobei die biologische Probe aus der Gruppe ausgewählt ist, die aus Blut, zerebraler Rückenmarksflüssigkeit, Zellen, einem Zellextrakt, einer Gewebeprobe und einer Gewebebiopsie besteht.

18. Verfahren nach einem der vorhergehenden Ansprüche, wobei das Filtrat einer DNA-Reinigung unterzogen wird und durch PCR, qPCR, digitale PCR, NGS, Massenspektrometrie oder Nanopore-Sequenzierung analysiert wird.

19. Verfahren nach einem der vorhergehenden Ansprüche, wobei das Filtrat einer DNA-Reinigung unterzogen wird, eine Sequenzierungsbibliothek nach dem schnellen Oxford-Nanopore-Bibliotheksaufbau-Verfahren erstellt und mit dem Oxford-Nanopore-GridlON-Sequenzer sequenziert wird.

20. Vorrichtung zur Verwendung in dem Verfahren nach einem der vorhergehenden Ansprüche, umfassend: ein oberes Gehäuse, einen Filter und ein unteres Gehäuse; wobei der Filter zwischen dem oberen Gehäuse und dem unteren Gehäuse angeordnet ist und aus dem polymermodifizierten Substrat hergestellt ist, wobei das Polymer des polymermodifizierten Substrats durch die Polymerisation von einem oder mehreren Monomeren mit der Struktur der Formel (1) hergestellt ist: 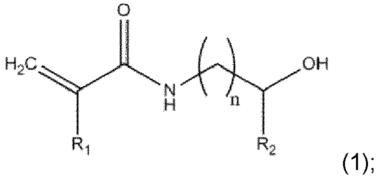 wobei R₁ unabhängig aus der Gruppe bestehend aus Wasserstoff, Methyl, Ethyl, Hydroxyl, C1-12-Alkyl, Phenyl ausgewählt ist; R2 unabhängig aus der Gruppe bestehend aus Wasserstoff, Methyl, Ethyl, C1-6-Alkyl, Amino, Phenyl ausgewählt ist; und n eine ganze Zahl von 1 bis 5 ist.

21. Vorrichtung nach Anspruch 20, wobei das obere Gehäuse der Vorrichtung mit einem Einlass versehen ist, während das untere Gehäuse mit einem Auslass versehen ist; wobei die biologische Probe durch den Einlass des oberen Gehäuses in die Vorrichtung eintritt, durch den Filter dringt und durch den Auslass des unteren Gehäuses aus der Vorrichtung fließt.

22. Verwendung einer Vorrichtung nach Anspruch 20 oder 21 zur Vorbereitung biologischer Proben für eine pathogene Untersuchung.

## Revendications

1. - Procédé d'enrichissement et de détection de microorganismes dans un échantillon biologique, comprenant les étapes suivantes : a) filtrer l'échantillon biologique à travers un substrat modifié par polymère, les cellules nucléées d'origine humaine dans l'échantillon étant capturées ou séparées par le substrat modifié par polymère et les microorganismes dans l'échantillon passant ou s'écoulant à travers le substrat modifié par polymère dans le filtrat ; et b) détecter les microorganismes présents dans le filtrat, les cellules nucléées comprenant un ou plusieurs des érythroblastes, leucocytes et cellules cancéreuses ; et le polymère étant préparé par la polymérisation d'un ou plusieurs monomères ayant la structure de formule (1): 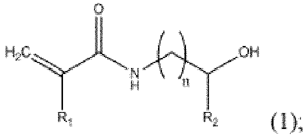 dans laquelle R₁ est indépendamment choisi dans le groupe constitué par hydrogène, méthyle, éthyle, hydroxyle, alkyle en C₁₋₁₂ , phényle ; R₂ est indépendamment choisi dans le groupe constitué par hydrogène, méthyle, éthyle, alkyle en C₁₋₆ , amino, phényle ; et n est un entier de 1 à 5.

2. - Procédé selon la revendication 1, dans lequel les cellules nucléées d'origine humaine sont des leucocytes.

3. - Procédé selon la revendication 1, dans lequel les microorganismes sont des bactéries.

4. - Procédé selon la revendication 1, dans lequel les microorganismes sont des champignons.

5. - Procédé selon l'une quelconque des revendications précédentes, dans lequel les érythrocytes dans l'échantillon passent ou s'écoulent à travers le substrat modifié par polymère dans le filtrat.

6. - Procédé selon l'une quelconque des revendications 1 à 4, dans lequel les plaquettes dans l'échantillon passent ou s'écoulent à travers le substrat modifié par polymère dans le filtrat.

7. - Procédé selon l'une quelconque des revendications 1 à 4, dans lequel les fibrinogènes dans l'échantillon passent ou s'écoulent à travers le substrat modifié par polymère dans le filtrat.

8. - Procédé selon l'une quelconque des revendications précédentes, dans lequel le monomère de formule (1) est, le N-hydroxyéthyl acrylamide, le N-(2-hydroxyéthyl) acrylamide, NHEMAA et le N-(2-hydroxyéthyl)acrylamide, HEAA.

9. - Procédé selon l'une quelconque des revendications précédentes, dans lequel le polymère comprend en outre un monomère supplémentaire, qui est le méthacrylate de butyle, et le monomère de formule (1) est copolymérisé avec le monomère supplémentaire pour former un copolymère.

10. - Procédé selon l'une quelconque des revendications précédentes, dans lequel le polymère a la structure de la formule (2) : 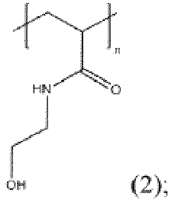 dans laquelle n est un entier de 10 à 50.

11. - Procédé selon l'une quelconque des revendications précédentes, dans lequel le polymère a la structure de formule (4) : 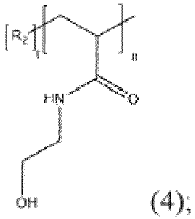 dans laquelle t est un entier de 50 à 90, n est un entier de 10 à 50, et R₂ est

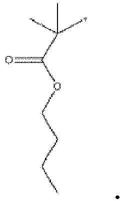

12. - Procédé selon l'une quelconque des revendications précédentes, dans lequel le polymère est un polymère segmenté.

13. - Procédé selon l'une quelconque des revendications précédentes, dans lequel le polymère est disposé sur le substrat par revêtement, pulvérisation ou imprégnation.

14. - Procédé selon l'une quelconque des revendications précédentes, dans lequel le substrat est le polypropylène, le polyéthylène téréphtalate, la cellulose, le polybutylène téréphtalate.

15. - Procédé selon l'une quelconque des revendications précédentes, dans lequel des éléments de surface du substrat modifié comprennent le carbone, l'oxygène et l'azote ; le pourcentage molaire total de carbone, d'oxygène et d'azote est défini comme étant de 100 %, le pourcentage molaire de carbone est d'environ 76,22 % à 79,84 %, le pourcentage molaire d'oxygène est d'environ 18,1 % à 21,04 %, et le pourcentage molaire d'azote est d'environ 2,05 % à 2,75 %.

16. - Procédé selon l'une quelconque des revendications précédentes, dans lequel le procédé est utilisé pour l'examen pathogénique d'échantillons biologiques.

17. - Procédé selon l'une quelconque des revendications précédentes, dans lequel l'échantillon biologique est choisi dans le groupe constitué par le sang, le liquide céphalo-rachidien, les cellules, un extrait cellulaire, un échantillon tissulaire et une biopsie tissulaire.

18. - Procédé selon l'une quelconque des revendications précédentes, dans lequel le filtrat est soumis à une purification d'ADN, et est analysé par PCR, qPCR, PCR numérique, NGS, MassSpec ou séquençage par nanopores.

19. - Procédé selon l'une quelconque des revendications précédentes, dans laquelle le filtrat est soumis à une purification d'ADN, une bibliothèque de séquençage est construite par le processus de construction rapide de bibliothèque d'Oxford Nanopore, et elle est séquencée avec le séquenceur GridION d'Oxford Nanopore.

20. - Dispositif destiné à être utilisé dans le procédé selon l'une quelconque des revendications précédentes, comprenant : un boîtier supérieur, un filtre et un boîtier inférieur ; dans lequel le filtre est situé entre le boîtier supérieur et le boîtier inférieur, et est fabriqué à partir du substrat modifié par polymère, dans lequel le polymère du substrat modifié par polymère est préparé par la polymérisation d'un ou plusieurs monomères ayant la structure de formule (1) : 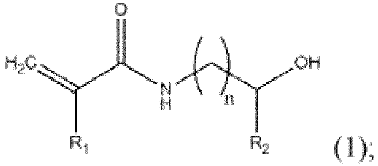 dans laquelle R₁ est indépendamment choisi dans le groupe constitué par hydrogène, méthyle, éthyle, hydroxyle, alkyle en C₁₋₁₂ , phényle ; R₂ est indépendamment choisi dans le groupe constitué par hydrogène, méthyle, éthyle, alkyle en C₁₋₆ , amino, phényle ; et n est un entier de 1 à 5.

21. - Dispositif selon la revendication 20, dans lequel le boîtier supérieur du dispositif comporte une entrée tandis que le boîtier inférieur comporte une sortie ; l'échantillon biologique entre dans le dispositif par l'entrée du boîtier supérieur, pénètre à travers le filtre, et s'écoule hors du dispositif par la sortie du boîtier inférieur.

22. - Utilisation d'un dispositif selon l'une des revendications 20 ou 21, pour préparer des échantillons biologiques en vue d'un examen pathogénique.
